# EUROPEAN PATENT SPECIFICATION

(11) **EP 2 486 121 B1**
(45) Date of publication and mention of the grant of the patent: **25.11.2015**
(21) Application number: 10763958.5
(22) Date of filing: 05.10.2010
(51) Int. Cl.: C12M 3/06

(54) **MICROSCALE MULTIPLE-FLUID-STREAM BIOREACTOR FOR CELL CULTURE**
MIKROSKALIGER BIOREAKTOR MIT MEHREREN FLÜSSIGKEITSSTRÖMEN FÜR ZELLKULTUREN
BIOREACTEUR A MULTIPLES COURANTS DE FLUIDE A MICRO-ECHELLE POUR LA CULTURE DE CELLULES

(30) Priority: 05.10.2009 US 573561
(43) Date of publication of application: 15.08.2012
(73) Proprietor: The Charles Stark Draper Laboratory, Inc., Cambridge, MA 02139-3563 (US)
(72) Inventor: CHAREST, Joseph, L., Cambridge MA 02138 (US); BORENSTEIN, Jeffrey, T., Newton MA 02464 (US)
(74) Representative: Somervell, Thomas Richard
(86) International application number: PCT/US2010/051461
(87) International publication number: WO 2011/044117

(56) References cited:
- WO-A2-2005/034624
- WO-A2-2006/052551
- WO-A2-2007/038572
- US-A1- 2002 166 585
- US-A1- 2005 148 064
- US-A1- 2006 019 326

## Description

### CROSS-REFERENCE TO RELATED APPLICATION

This application claims priority to and the benefit of, and incorporates herein by reference in its entirety, co-pending U.S. Patent Application No. 12/573,561, which was filed on October 5, 2009.

### FIELD OF THE INVENTION

The present invention relates, generally, to systems and methods for culturing cells. More particularly, various embodiments relate to multi-layer microfluidic cell culture devices having multiple intercommunicating channels, and to methods of using such devices for the controlled culture of kidney and other cells.

### BACKGROUND

Kidney disease presents a serious health risk in the United States. Approximately one out of nine adult Americans suffers from chronic kidney disease (CKD), and approximately 450,000 patients have end-stage renal disease (ESRD). Current standard-of-care treatments, such as hemodialysis and hemofiltration, provide assistance to the damaged organs, but typically do not directly facilitate replacement or regeneration of diseased tissues. Modern renal tissue engineering and regenerative strategies, on the other hand, seek to replace or repair kidney tissues using viable kidney-specific cells and cell-biomaterial constructs. Culturing kidney-specific cells, progenitor cells, or stem cells generally requires tight control of chemical, biological, and biophysical cues to ensure proper cell growth and phenotypic function.

Conventionally, kidney-related cells have been cultured in plates, dishes, and flasks, and cell media and other fluids are delivered to the cells manually and left in a static state. More recent cell culture methods utilize bioreactors with multiple chambers, tubular structures, or hollow fibers to establish a fluid flow that facilitates exposing the cells to controlled levels of shear stress or to various chemical environments. However, these methods generally do not, or only to very limited extent, permit exposing the cells simultaneously to multiple user-specified chemical, biological, and biophysical stimuli. Further, they typically do not mimic the conditions and fluidic structure that kidney cells experience in vivo, such as close proximity of several cell phenotypes and a microscale vascular-like structure. Since the conditions that cells experience influence how they function, these methods may, therefore, fail to induce the desired functions of the cell types, or induce undesired functions. Further, these methods typically do not allow for the culturing of multiple cell types at distinct locations within the bioreactor structure. To improve kidney-related cell culturing for medical applications, it is thus desirable to provide systems and methods for growing multiple cell types under controlled culture conditions. International application WO2005/034624 describes a 3-dimensional system for engineering tissues containing multiple cell types, wherein individual layers of the system comprise channels divided longitudinally into two compartments by a centrally positioned membrane, and wherein each compartment can comprise a different cell type.

### SUMMARY

The present invention provides, in various embodiments, microfluidic multi-channel bioreactor devices for culturing cells in an environment that controls multiple chemical, biological, and biophysical parameters, thereby facilitating, for example, better simulation of in vivo conditions. Such bioreactor systems may, for example, include two polymer .layers separated by a permeable or semi-permeable membrane. Each layer defines one or more microchannels, which are, in operation, filled with a fluid such as, e.g., buffer solution, cell culture medium, blood, or urine. Fluid flow may be induced in a channel, e.g., by applying a pressure difference between the channel inlet and outlet.

Channels in one layer may "communicate" with one or more channels in the other layer through the membrane. Communication, as the term is used herein, refers to any type of interaction between the channels, whether chemical, physical (e.g., thermal, mechanical, or fluid-mechanical) or biological in nature. For example, communication may involve fluid communication, i.e., the transport of fluid or components thereof between the channels, or a mechanical interaction, such as, i.e., the conferment of pressure in one channel onto the other channel. Channels within the same layer may also communicate with each other through the membrane and a channel in the other layer that overlaps geometrically with both of them. Geometric overlap of channels herein connotes that projections of the channels into a plane parallel to the membrane (or locally parallel to the membrane in cases where the layers and membrane are not flat) overlap, even though the channels do not occupy the same physical space.

The degree of communication between channels in one layer depends, among other factors, on the distance between the channels and on the height and width of their cross-sections. Thus, by varying a subset or all of these parameters along the length (i.e., along the longest dimension or axis) of the channels, the level of communication can be controlled as a function of the position along the channels. Such control over the communication between the channels, in turn, facilitates control over fluid-mechanical and chemical parameters along the channels via the injection of fluids and the control of flow rates and pressures at the input and output ports. For example, if two fluids of different composition are injected into neighboring channels at the inlet, the two fluids may mix due to chemical communication, i.e., mass transfer, between the channels and result in a third, mixed composition at the outlets. Similarly, different pressures may be applied at the ports (inlets and/or outlets) of two channels, and result in profiles of fluid-mechanical parameters along the channel length that are determined (at least in part) by the geometries of the individual channels and the level of mechanical communication therebetween.

Microfluidic devices as described above may be advantageously employed for the culturing of cells. The channels may be populated with cells of a single or of multiple types, which may be placed at distinct locations within the channels. The relative placement and shape of the channels, the cell location inside the channels, and operational parameters such as fluid compositions and pressures applied to the ports collectively afford an unprecedented level of control over the microenvironment of the cultured cells and the administration of chemical, biological, mechanical, and biophysical signals to the cells. With control over these parameters, the bioreactor may be utilized to influence cell function and to facilitate culture of multiple cell types at distinct locations within the bioreactor structure. A user may administer any combination of parameters, simultaneously or in time according to a specific scheme, in order to alter cell function in a desired fashion and/or for a particular purpose. Cell functions that may thus be tailored include, but are not limited to, the enhancement or limitation of proliferation, the maintenance of stem cell pluripotency, or the differentiation of cells towards a specific phenotype. Cells may also be directly engineered.

Embodiments of the invention provide a platform to culture and develop cell populations for a variety of applications. For example, cell cultures may be tailored for a specific therapeutic application, e.g., a new regenerative therapy or the enhancement of a previous cell-based therapy. In some embodiments, the bioreactor is designed to mimic a natural organ, and used to study cell development and organ functions. The bioreactor device may also be a precursor to, or part of, a bioengineered artificial organ. Different applications may, generally, make use of different aspects and features of the invention.

In one aspect, the invention provides a microfluidic bioreactor device that includes a first polymer layer defining first and second microchannels therein and a second polymer layer defining a third microchannel therein. A membrane separates the first and second channels from the third channel (and, optionally, a fourth channel) at geometrically overlapping portions while permitting communication (e.g., fluid communication or mechanical communication) between the overlapping portions of the microchannels. The first and second channels may communicate with each other via the third channel. At least one geometric parameter of at least one of the microchannels varies along a length of the channel within the overlapping portion(s). Geometric parameters that may vary along the microchannel length include the distance between the first and second channels, and/or the width and/or depth of any of the channels.

In some embodiments, the polymer layers include or essentially consist of a biopolymer. The membrane, or a portion thereof, may be semi-permeable, and may be formed by a porous or semi-bulk-permeable material. In certain embodiments, the membrane includes or consists essentially of fleece, micromolded polydimethylsiloxane (PDMS) or other silicone polymer, polyethersulfone, an electrospun material, or a tracked-etched membrane.

The term "bioreactor device," as used herein, refers to the microfluidic structure described above, whether in use or not. In embodiments configured for cell culture, the bioreactor device further includes cells in at least some of the microchannels. For examples, the cells may be kidney cells, and the microchannels may further be configured so as to collectively mimic kidney tissue. The cells may adhere to the membrane or the walls of the channels, and/or may be suspended in fluid contained in the channels. The cells may comprise multiple types, which, in some embodiments, vary along the length of the channel.

The device may further include a fluid, such as, for example, cell culture medium, buffer solution, blood components, whole blood, urine, dialysate, water, or a filtrate in the microchannels. In certain embodiments, the fluid is or includes a solution mimicking a bodily fluid. The fluids in the channels may have fluid mechanical parameters (e.g., pressure, flow rate, shear rate, viscosity, etc.) associated with them. The values of a fluid mechanical parameter may differ substantially (e.g., by a factor of greater than 1.1, greater than 1.5, greater than 2, or greater than 10) between any two microchannels. For example, they may differ between the first or second channel (which may be formed in a first layer) and the third channel (which may be formed in second layer), and/or between the first and second channels and/or the third and fourth channels, Moreover, concentrations or concentration gradients of a constituent in the fluids may vary between any two channels on the same or one different sides of the membrane. The geometric parameter(s) of the microchannels may vary gradually along the channel length. In some embodiments, one or more parameters vary based on a predetermined fluid-mechanical profile in at least one of the microchannels in the first polymer layer. The fluid-mechanical profile may be, for instance, a connective or diffusive transport profile, or a shear stress profile. In some embodiments, the variation of the geometric parameter(s) facilitates variance of at least one of a chemical or a mechanical stimulus along the length of that microchannel.

In another aspect, various embodiments of the invention are directed to a method of culturing cells by providing a bioreactor as described above, introducing cells into at least one of the microchannels, and culturing the cells. The method may also include introducing a fluid into at least one of the microchannels. In some embodiments, the method further involves exposing the cells to a mechanical, a chemical, and/or a biological stimulus and, optionally, measuring a response of the cells (e.g., a change in the cell function) to the stimulus. The cells may be seed in the microchannel(s) at selected locations, and cells different locations may be exposed to different stimuli. Moreover, different cell types may be introduced into the microchannel(s). In some embodiments, different types of cells are introduced into different microchannels, and in some embodiments, different types of cells are seeded at different locations within the same microchannel.

In yet another aspect, the invention provides a method for mimicking a kidney. The method involves providing a bioreactor having the features described above, in which the parameter varies in a way that mimics a kidney structure. The method further involves introducing kidney cells into at least one of the microchannels and culturing the cells. The bioreactor may than be used, for example, extracorporeally in renal therapy. In some embodiments, the bioreactor may be implanted into a patient.

These and other objects, along with advantages and features of the embodiments of the present invention herein disclosed, will become more apparent through reference to the following description, the accompanying drawings, and the claims. Furthermore, it is to be understood that the features of the various embodiments described herein are not mutually exclusive and can exist in various combinations and permutations.

### BRIEF DESCRIPTION OF THE DRAWINGS

In the drawings, like reference characters generally refer to the same parts throughout the different views. Also, the drawings are not necessarily to scale, emphasis instead generally being placed upon illustrating the principles of the invention. In the following description, various embodiments of the present invention are described with reference to the following drawings, in which:
FIGS. 1A-1C are schematic perspective views of a microfluidic device in accordance with one embodiment of the invention;
FIG. 2 is a scanning electron microscopy (SEM) image of a cross-section of the device shown in FIGS. 1A-1C;
FIGS. 3A-3C are schematic top-views of channels whose spacing, width, and depth, respectively, varies along a length of the channels in accordance with various embodiments of the invention; and
FIGS. 4A-4D are schematic side views of a device having a channel in one layer communicating with three channels in another layer in accordance with one embodiment of the invention, which illustrate the effect of a variation in channel distance, width, and depth, respectively, on the level of communication.

### DESCRIPTION

The invention relates, in various embodiments, to microfluidic devices featuring two channel-containing layers separated by a membrane through which microchannels in one layer can communicate with microchannels in the other layer. The channel-containing layers are typically formed of a polymer, but the invention is not limited in this way. In some embodiments, the layers may consist of or include ceramics, metal, glasses, or other non-polymer materials. Suitable non-degradable polymer materials include polystyrene, PDMS, polycarbonate, and polyurethane. For certain applications, the use of biodegradable or biocompatible materials, such as polyglycerol sebacate, polyesteramide, polyoctanediol citrate, polydiol citrate, silk fibroin, or polycaprolactone may be advantageous. Further, in certain embodiments, biopolymers, such as, e.g., proteins or gels, may be used.

The channel-containing layers may have thicknesses ranging from less than 100 microns to several millimeters. The membrane, on the other hand, is only approximately 1 micron to approximately 100 microns thick. To allow mass transfer across the membrane, the membrane, or at least a portion thereof, is permeable or semi-permeable (i.e., selectively permeable to some, but not to other ions and molecules, depending upon physical or chemical properties of the molecule and the membrane). Permeability may be achieved by using a semi-porous or porous material (such as polyethersulfone), in which mass transfer takes place through the pores, or a bulk-permeable material (such as PDMS or a fleece-like material). In certain embodiments, the membrane is created by electrospinning a polymer onto one of the channel-containing layers-a process which results in a flexible, porous polymer mesh.

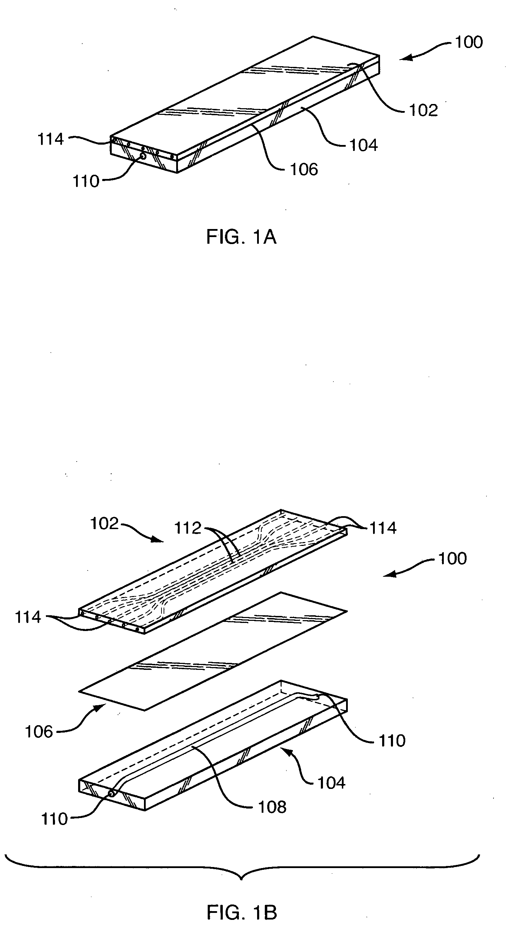

A simple exemplary microfluidic device is schematically illustrated in FIGS. 1A-1C. While FIG. 1A is an assembled view of the device 100, FIG. 1B is an exploded view of the device 100 that illustrates the two polymer layers 102, 104 and the membrane 106 separately. In this example, the bottom layer 104 defines a single channel 108 extending along the layer 104 and being accessible through ports 110 at both ends. The top layer 102 includes five channels 112, each having smaller diameters than the channel 108 in the bottom layer 104. These channels 112 are closely spaced and laterally overlap with the wider channel 108 along the larger portion of the channel length, but diverge at the ends of the channels 112 to increase the convenience of accessing them through their respective ports 114.

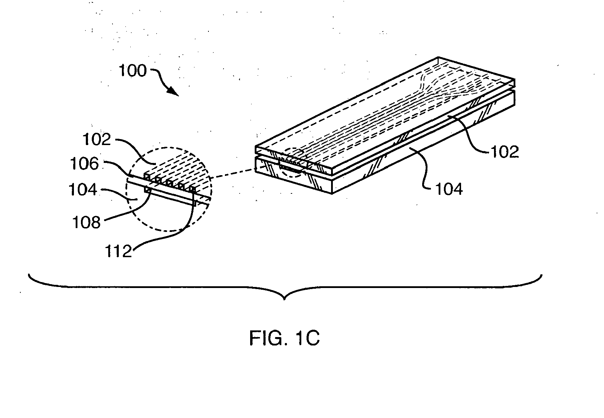

FIG. 1C provides a translucent view of the assembled device 100, along with an enlarged view, in the inset, of a cross section showing the membrane 106 and polymer layers 102, 104 slightly spaced apart. As can be seen from the inset, the channels 108, 112 defined in each of the polymer layers 102, 104 each have an open side facing the membrane 106. As a result, the channels 112 in the upper layer 102 and the wide channel 108 in the bottom layer 104 can interact via the membrane 106. In many embodiments, the channels 108, 112 have rectangular cross-sections, as shown in FIG. 1C. In general, however, channel cross-sections may take any polygonal or rounded shape. For example, channel cross-sections may be semicircular, having their straight boundary formed by the membrane 106.

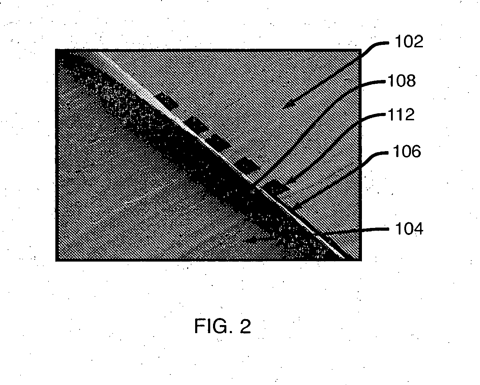

An SEM image of a cross-section of a device 100 having the basic structure illustrated in FIGS. 1A-1C is shown in FIG. 2. The porous layer 106 has a thickness of about 6 µm. The five microchannels 112 in the top layer 102 are each approximately 50 µm wide and approximately 50 µm deep. In general, typical embodiments have channel depths ranging from a few microns to hundreds of microns, and widths ranging from a few microns to a few millimeters.

In one embodiment, during operation of a device 100, such as the one shown in FIGS. 1A-1C and 2, fluid, which may include cells, is conveyed to the microchannels 108, 112 through the ports 110, 114. Since each channel has distinct inlet and outlet ports, the type of fluid and flow conditions may be controlled independently for each channel. Consequently, fluid mechanical parameters in one channel may differ from fluid mechanical parameters in another channel, whether the other channel is in the same or another layer. Fluid mechanical parameters include, for example, pressure, flow rate, shear rate, shear stress, laminarity, and viscosity. Other parameters that may differ between channels 108, 112 include temperature, thermal conductivity, electrical conductivity, density, and chemical composition of the fluid. Further, any combination of parallel flow in multiple channels 108, 112, counter flow, flow across the porous layer 106, and static conditions may be employed. Parameters may be selected for a specific application. For example, to mimic the Loop of Henle, a portion of the kidney where water and salts are reclaimed from a waste stream and concentrated urine is formed, a counter flow between two neighboring channels that are fluidically connected at one end may be established. To mimic a glomerulus, where blood filtering takes place, blood may be flown through one channel, while dialysate may be flown through an adjacent channel. The simulation of processes like, e.g., blood filtration using the devices described herein exploits the ability of channels 108, 112 to communicate with each other through mass transfer, pressure equilibration, or otherwise.

The degree of communication between channels on both sides of the membrane 106, as well as between channels in the same layer via a channel on the other side of the membrane 106, depends on various geometric parameters, including the proximity of the channels and the dimensions of their cross-sections, as well as on the properties (e.g., material, thickness, pore size, etc.) of the membrane 106. Further, communication may be influenced by concentrations and concentration gradients of various fluid constituents.

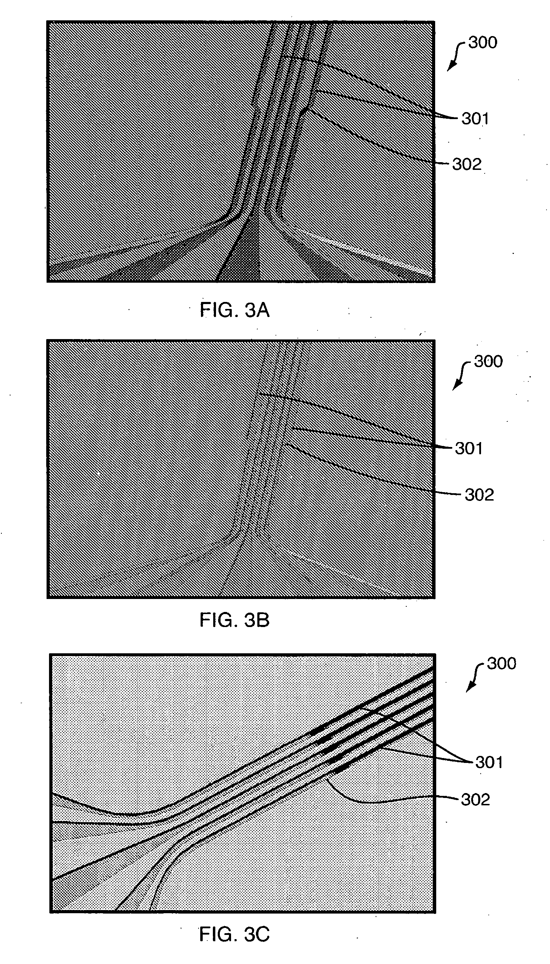

While the geometry of the channels 108, 112 depicted in FIGS. 1A-1C and 2 does not change along the length of the channels 108, 112, this need not be the case. Rather, various embodiments of microfluidic bioreactors may utilize variations of one or more of these parameters to control communication between the channels and, consequently, conditions within the channels, as a function of the position along the channel length. FIGS. 3A-3C illustrate, in top view, a device layer 300 having five microchannels 301 whose geometric properties change in a step-like manner at one point 302 along the channel 301. In FIG. 3A, the distance of the two outermost channels 301 to the inner channels 301 changes abruptly. In FIG. 3B, the two outmost channels 301 get wider and the neighboring two inner channels 301 become narrower, while the center channel 301 does not change its cross-section. Finally, in FIG. 3C, the depth of the channels 301 changes at point 302.

The geometry may vary in more than one location. Further, it may very gradually. For example, the distance between two straight channels may increase linearly along a length of the channels. Since, in that case, the channels are no longer parallel, the variation in the relevant geometric parameter (in the example, a distance) may be defined with respect to a length or axis of either one of the channels, a symmetry axis between the channels, or, generally, any geometric axis along which both channels have a projection component (i.e., which is not perpendicular to any of the channels). The phrase "along a length of the channel" is meant to encompass all these possible reference axes. To ensure communication between diverging channels via another channel in the layer on the other side of the membrane on the shortest possible route, that other channel will typically vary in width accordingly (e.g., have a trapezoidal projection into the layer if the intercommunicating channels in the other layer diverge linearly). Alternatively, the wider channel that provides the means of communication may have a width equal to at least the largest distance between the narrower channels in the relevant portion of the device. (Note that the divergence of the five microchannels 112 in FIGS. 1A-1C near the ports 114 is distinguished from the change of the geometric parameter described above in that, beyond the region where the five channels 112 are parallel, they do not overlap with the wider channel 108 in the bottom layer 104.)

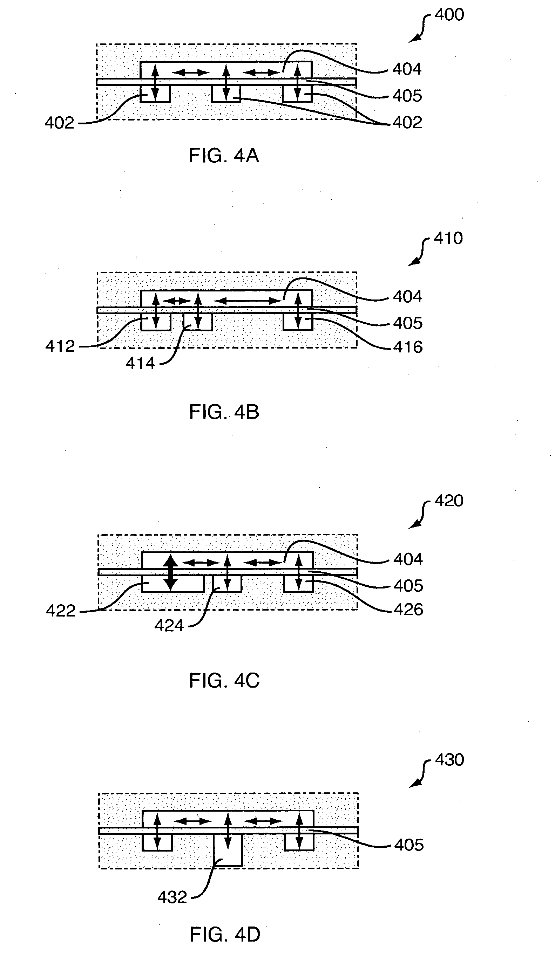

The effect of channel geometry on the interactions between channels, and in particular on the interaction between channels in the same layer, across the membrane, and through a laterally overlapping channel in the other layer, is illustrated schematically in FIGS. 4A-4D. The initial configuration 400, comprising three approximately equidistant narrow channels 402 having approximately the same cross-sections and a wider channel 404 in the upper layer that is flush with the two outer channels 402 in the lower layer, is shown in FIG. 4A. Due to the characteristics of microfluidic devices, particularly the predominance of laminar flow in the channels, mixing of solutions across fluids streams within the channels is largely dominated by diffusion. Since diffusion is dependent upon path length, changing channel geometry to alter path lengths of diffusion between channels and within channels provides a means to control the delivery of soluble constituents to various regions along the length of the channels. In addition, diffusion depends on cross-sectional area, so altering channel width or depth can influence the amount of diffusion across the membrane.

In the cross-sections of FIGS. 4A-4D, the amount of diffusion across the membrane 405 is indicated by the width of vertical arrows, and diffusion path lengths across the width of the channel 404 in the upper layer are denoted by horizontal arrows. Decreasing the distance between channels in the bottom layer reduces the diffusion path length through the top channel, resulting in increased diffusion between the two channels in the bottom layer. Thus, in the configuration 410 shown in FIG. 4B, diffusion transport between two closely spaced channels 412, 414 is greater than diffusion transport between the two more distantly spaced channels 414, 416. In the configuration 420 shown in FIG. 4C, diffusion across the membrane 405 is increased for the wider channel 422 in the bottom layer due to the enlarged contact area with the membrane 405. Consequently, diffusion between the wider channel 422 and each of the narrower channels 424, 426 is likewise increased. In the configuration 430 shown in FIG. 4D, the altered channel depth of the central channel 432 influences the diffusion path length within the channel 432, which changes the extent of communication by increasing the total channel volume for a given amount of diffusion.

In embodiments where the geometric parameters vary along the channel length, the diffusion-based delivery of fluid constituents (e.g., blood components, nutrients, or pharmaceutical agents) can be tuned via channel geometry for different regions of the channels. This is particularly useful for culture of kidney cells, as cells along a kidney tubule, for example, experience varying chemistries dependent upon their location within the path of the tubule. Thus, variations in the geometry of the channels provide a unique way to culture kidney cells in a microfluidic device such that they experience conditions similar to those experienced in vivo.

Microfluidic devices in accordance with embodiments of the invention may be manufactured using various techniques known in the art, including replica molding, conventional machining, injection molding, or solid freeform fabrication. To produce the individual polymer layers by means of replica molding, a master mold featuring a negative relief of the desired structure is fabricated for each layer. Widely used methods of creating the master mold include soft lithography, wet etching, plasma etching, and electroplating.

Producing the master mold by soft lithography, for example, involves designing a photomask that defines the ridges of the master mold, corresponding to the indentations of the final layer, as transparent regions in an otherwise opaque sheet. The mask layout may be defined in a computer drawing, and may then be converted, e.g., with a software package such as Tanner L-Edit, into a Computer-Aided Design (CAD) layout, which is suitable for subsequent physical writing of the mask by electron-beam lithography or a similar technique.

As another preparatory step, a substrate wafer, e.g., made from silicon, may be spin-coated with a viscous solution of a suitable photoresist, such as, for example, SU-8. Typically, the wafer is spun rapidly, at 1200 to 4800 revolutions per minute, for a time duration ranging from several tens of seconds up to minutes, to produce a uniformly thick layer of photoresist with a thickness of up to tens or even hundreds of micrometers. Then, the photomask may be placed on the wafer, and the photoresist in the transparent regions of the mask may be chemically stabilized by exposure to UV light. Photoresist in non-exposed regions may be subsequently removed by exposure to a chemical developing agent, and the remaining photoresist may be hardened at elevated temperatures to form a durable negative relief. In an etching step, a chemical agent may be employed to remove the upmost layer of the substrate in regions that are not protected by photoresist, generating a channel pattern in negative relief in the wafer, which now constitutes the master mold. The photoresist, no longer needed, may afterwards be removed from the substrate. Next, a liquid polymer may be cast into the master mold, cured, and peeled off, resulting in a replica mold of the channel-containing layer of the device (e.g., layer 102 or 104).

The membrane that separates the channel(s) in one layer from those in the other layer may be fabricated by coating a suitable material onto a wafer, curing it, if applicable, and peeling it off. Another fabrication approach involves electrospinning of a membrane with desired porosity, thickness, and other desired properties. Alternatively, a commercially available membrane (e.g., a track-etched polycarbonate membrane from Sterlitech, Kent, Washington) or a membrane fabricated in situ may be used. The polymer layers and membrane may then be assembled, aligned, and plasma-bonded or otherwise temporarily or permanently attached to each other.

The techniques described above provide great versatility in producing channels of desired width, depth, flow path, and configuration. For example, channels may be designed to travel along curved paths, branch into multiple channels, or form complex networks. Thus, the devices may be adjusted to mimic important features of biological organs and tissues, such as, e.g., a microvasculature or a portion of a nephron. In some embodiments, the channels are designed based on a desired fluid-mechanical profile, such as a profile defined by convective or diffusive transport properties or other fluid-mechanical parameters as a function of the position in the channel(s). Alternatively or additionally, channel design may be based on a desired concentration profile of certain constituents in fluid to be injected into the device. Computational models may be employed to calculate, from a given fluid-mechanical or concentration profile, the geometry and configuration of the channels that would yield such a profile. The profile may be chosen to mimic a particular biological organ, or to optimize a biologically relevant parameter. For example, clearing a channel of small molecules may be optimized by making the channel appropriately shallow. Alternatively, the mechanical, chemical, or biological microenvironment within a channel or compartment may be optimized to induce conditions suitable for increasing cell viability.

Various modifications and additional features may be implemented in microfluidic bioreactor devices in accordance with the invention. In some embodiments, both layers include multiple channels. For example, one layer may contain two wider channels, each of which laterally overlaps with and establishes communication between two of four narrower channels in the other layer. In another exemplary configuration, each layer may contain a channel that laterally overlaps with multiple channels in the other layer. In certain embodiments, channels within one layer communicate not only via a channel on the other side of the membrane, but also directly through a fenestrated side wall or a porous or otherwise permeable material separating the channels in that layer. Further, a device may include more than two layers, which may be integrated by a header component that fluidically connects the ports of the various layers. Thus, a three-dimensional network may be produced. The channels themselves may feature smooth walls, smooth bifurcations, distensible walls and/or nearly perfect circular or semicircular cross sections.

In certain embodiments, a bioreactor that may otherwise be structurally and functionally similar to the ones described above may be produced from a single polymer layer or block. Channels may be drilled into the block at various heights, e.g., so that the center axes of two channels come to lie in one plane, and the center axis of a third channel comes to lie in another, parallel plane. In certain portions, the material between the channels in the two planes may be partially etched away or otherwise removed so that a membrane-like structure is formed between the channels. Alternatively, one or more channels with a vertical dimension equal to two desired channel depths may be formed in the block, and a membrane may be inserted to split the channel(s) into vertically stacked channels.

To prepare the device for cell cultures, the chemical characteristics of the channel walls may optionally be adjusted by flushing the channels with suitable solutions, such as bovine serum albumin (BSA) or a surface-functionalizing solution. Depending upon the application, proteins typically found in extracellular matrix (ECM), such as collagen, laminin, fibronectin, or elastin, may be attached to the walls via surface functionalization methodologies.

The micro-device may then be incorporated into an experimental setup by fitting tubing to the inlets and outlets, injecting fluid which may contain cells, and/or connecting the device to other apparatuses. Cells may float in the fluid that is contained in or flows through the channels. Alternatively or additionally, the cells may adhere to the walls and/or membrane. In membranes with sufficient pore sizes, the cells may occupy the pores themselves, thus forming a porous cell culture layer. Thus, the cells may be exposed to stimuli from fluids on both sides of the membrane.

In various embodiments, multiple types of cells are cultured in the bioreactor. Different types may be co-cultured in the same location, injected into different channels, or seeded at different locations within the same channel. In general, the adhesion of cells to the walls and membrane depends on the surface chemistry, which may be specific to binding sites found on some, but not on other cell types. Thus, targeting different locations within the channel with different cell types may be accomplished by changing the surface chemistry along the channel length, e.g., by micropatterning surface adhesion molecules, or creating a nanotopography on the channel wall. Additional factors that influence where particular cell types are cultured include the order in which cell types are introduced into the channel and the orientation in which the device is held during cell seeding (which may exploit gravitational forces to place cells at particular locations)..

The cell cultures may be exposed to multiple user-imposed chemical, physical, biological, and/or biophysical stimuli. For example, the channel configuration may allow both normal-pressure and shear-stress biophysical stimuli. Shear stress may be regulated by controlling the flow rate through the channels, while pressure within the channels may be set by the externally supplied pressure. Pressure may be controlled for multiple channels on either side of the porous layer. Since multiple channels may be employed on each side of the membrane, the pressure across the membrane (and thus, in some embodiments, across the porous cell layer) can be controlled for multiple locations independently. Pressure control across a cell layer is of particular importance for mimicking conditions of cells that perform transport functions in vivo, such as the various cells found in the kidney.

Controllable biological parameters include the density and types of cells that surround a particular cell culture. Cells in the porous layer may interact with one set of cell species on one side, and another set of species on the other side. In some embodiments, the microscale width of the channels limits the number of cells in an area, thereby limiting cell-cell interactions. Further, the microscale depth of the channels may influence the level of communication between cells on the porous layer and cells on the channel wall opposite that layer. Collectively, these effects provide an avenue for the user to control cell-cell interactions via the local channel geometry. Further, as mentioned previously, the variation of channel proximity, width, and depth along the channel length facilitates control over the chemical environment along the length of the channel, which may be used to approximate the changes in the chemical environment that cells may experience in vivo, e.g., along the length of a kidney tubule. Since cell type and function change along the length of the kidney tubule, providing a varied chemistry may support the function of those cells when cultured in the bioreactor.

The cells may interact not only with each other, but also with the fluid in the channel. For example, cell proliferation may change in response to a particular drug, a change in the nutrient content of the fluid, the pH of a buffer solution, etc. Cells may also absorb or secret certain compounds, thus changing the fluid composition. In one embodiment, whole blood may flow through a channel on one side of the membrane, and a dialysate may flow through the channel on the other side of the channel, mimicking counter flow in the kidney and inducing cells to filter the blood along its way.

The effect of various stimuli on the cells may be observed and/or measured in several ways. Optical microscopy may be used to detect any change in the sizes or shapes of the cells. Fluid may be diverted at the outlet and analyzed for certain constituents to determine the secretion or absorption of certain compounds, which may provide information about cellular metabolism and/or protein expression. Measurements of the rate at which fluid, particles, and molecular species pass through the membrane, of pressure across the membrane, or of the electrical resistance across the membrane may be used to assess how well the cells block the membrane pores.

As will be appreciated by those of ordinary skill in the art, microfluidic bioreactors as described herein provide a platform for multiple research and medical applications, in particular, as they relate to cultures of kidney-related cells or stem cells. The control of various parameters facilitates culturing cells in a prescribed environment in order to elicit a desired cell function. For example, stem cells may be cultured for a particular therapeutic application, while maintaining potency or encouraging differentiation to a certain phenotype.

The bioreactor devices also improve the capabilities of studying cell function in vitro, yet under conditions closely resembling those in vivo. Because the environmerital parameters in the bioreactors are well-controlled, the observation of cell response to known inputs for a given set of conditions is possible. In certain embodiments, the bioreactor, populated with kidney cells, mimics important functional components of the kidney. Thus, pharmaceuticals for kidney therapy may be developed on a well-controlled platform. Drug efficacy and toxicity may be tested safely in vitro by evaluating kidney-related cells in the bioreactor for viability and markers of cell health after they have received a pharmaceutical insult. The high level of control over multiple parameters may reduce experimental variability and improve accuracy, as compared to traditional methods of drug testing.

Certain embodiments of bioreactor devices may be used for the treatment of end-stage renal disease. The bioreactor and adherent cells may be configured such that the device performs some functions of the kidney, thereby serving as a kidney-assist device, which may be used extracorporeally in renal replacement therapy. The device may also be employed as a scaffold for tissue engineering. Specifically, by providing a controlled architecture mimicking structures of the kidney and allowing for the attachment of cells, the bioreactor may serve as a scaffold to generate kidney specific tissue constructs. Such constructs may be implanted into the body of a patient to replace diseased kidney tissue.

## Claims

1. A microfluidic bioreactor device comprising:
a first polymer layer defining first and second microchannels therein and a second polymer layer defining a third microchannel therein; and
a membrane separating the first and second channels from the third channel at geometrically overlapping portions therebetween, the membrane permitting communication between the overlapping portions of the microchannels,
wherein a distance between the first and the second microchannels varies along a length thereof and a depth and a width of the first microchannel and a depth and a width of the second microchannel remain constant along the length thereof within the overlapping portions.

2. The device of claim 1 wherein the first microchannel communicates with the second microchannel via the third microchannel.

3. The device of claim 1, wherein the membrane comprises at least one of a fleece, an electrospun material, micromolded polydimethylsiloxane, polyethersulfone, or a track-etched membrane.

4. The device of claim 1, further comprising at least one type of cell that adheres to at least one of: the membrane and one wall of the microchannels.

5. The device of claim 4, wherein the type of cell varies along a length of the microchannel.

6. The device of claim 1, further comprising a fluid in the microchannels, wherein the fluid consists of at least one of cell culture medium, buffer solution, blood components, whole blood, urine, dialysate, water, filtrate, or a solution mimicking a bodily fluid.

7. The device of claim 1, wherein a value of a pressure, a flow rate, a shear rate, or a viscosity in at least one of the first and second microchannels is substantially different from a value of a pressure, a flow rate, a shear rate, or a viscosity in the third microchannel.

8. The device of claim 1, further comprising a fourth microchannel on the same side of the membrane as the third microchannel, a value of a fluid mechanical parameter in the fourth microchannel being substantially different from a value of a fluid mechanical parameter is the third microchannel.

9. The device of claim 1, wherein a concentration or a concentration gradient of a constituent in the fluid in at least one of the first and second microchannels is substantially different from a concentration or a concentration gradient of the constituent in the fluid in the third microchannel.

10. The device of claim 1, wherein the distance between the first and second microchannels varies gradually along the length thereof.

11. The device of claim 1, wherein the variation of the distance between the first and second microchannels along the length thereof facilitates variance of at least one of a chemical or a mechanical stimulus along the length of that microchannel.

12. A method of culturing cells, the method comprising:
(a) providing a bioreactor comprising a device of any of the claims 1 to 13;
(b) introducing cells into at least one of the microchannels; and
(c) culturing the cells.

13. The method of claim 12, further comprising introducing a fluid into at least one of the microchannels.

14. The method of claim 12, further comprising exposing the cells to at least one of a mechanical, a chemical, or a biological stimulus; and measuring a change in cell function of the cells to the stimulus.

15. The method of claim 12, wherein step (b) comprises:
introducing different types of cells into the at least one microchannel;
introducing different types of cells into different microchannels; and
seeding different types of cells at different selected locations within the at least one microchannel.

## Patentansprüche

1. Mikrofluidische Bioreaktorvorrichtung umfassend:
eine erste Polymerschicht, die einen ersten und einen zweiten Mikrokanal darin definiert, und eine zweite Polymerschicht, die einen dritten Mikrokanal darin definiert; und
eine Membran, die den ersten und zweiten Kanal von dem dritten Kanal in geometrisch sich überlappenden Abschnitten dazwischen trennt, wobei die Membran eine Kommunikation zwischen den sich überlappenden Abschnitten der Mikrokanäle gestattet,
wobei ein Abstand zwischen dem ersten und dem zweiten Mikrokanal einer Länge davon entlang variiert und eine Tiefe und eine Breite des ersten Mikrokanals und eine Tiefe und eine Breite des zweiten Mikrokanals der Länge davon entlang innerhalb der sich überlappenden Abschnitte konstant bleiben.

2. Vorrichtung nach Anspruch 1, wobei der erste Mikrokanal mit dem zweiten Mikrokanal über den dritten Mikrokanal kommuniziert.

3. Vorrichtung nach Anspruch 1, wobei die Membran mindestens eines umfasst von einem Vlies, einem elektrogesponnenen Material, einem mikrogeformten Polydimethylsiloxan, Polyethersulfon oder einer spurengeätzten Membran.

4. Vorrichtung nach Anspruch 1, ferner mindestens einen Typ Zelle umfassend, die an mindestens einer anhaftet von: der Membran und einer Wand der Mikrokanäle.

5. Vorrichtung nach Anspruch 4, wobei der Typ Zelle einer Länge des Mikrokanals entlang variiert.

6. Vorrichtung nach Anspruch 1, ferner ein Fluid in den Mikrokanälen umfassend, wobei das Fluid aus mindestens einem besteht von Zellkulturmedium, Pufferlösung, Blutkomponente, Ganzblut, Urin, Dialysat, Wasser, Filtrat oder einer ein Körperfluid nachahmenden Lösung.

7. Vorrichtung nach Anspruch 1, wobei ein Wert von einem Druck, einer Strömungsrate, einer Scherrate oder einer Viskosität in mindestens einem von dem ersten und zweiten Mikrokanal im Wesentlichen von einem Wert von einem Druck, einer Strömungsrate, einer Scherrate oder eine Viskosität im dritten Mikrokanal verschieden ist.

8. Vorrichtung nach Anspruch 1, ferner einen vierten Mikrokanal auf derselben Seite der Membran wie der dritte Mikrokanal umfassend, wobei ein Wert eines fluidmechanischen Parameters im vierten Mikrokanal im Wesentlichen von einem Wert eines fluidmechanischen Parameters in dem dritten Mikrokanal verschieden ist.

9. Vorrichtung nach Anspruch 1, wobei eine Konzentration oder ein Konzentrationsgradient eines Bestandteils in dem Fluid in mindestens einem von dem ersten und zweiten Mikrokanal im Wesentlichen von einer Konzentration oder einem Konzentrationsgradienten des Bestandteils in dem Fluid im dritten Mikrokanal verschieden ist.

10. Vorrichtung nach Anspruch 1, wobei der Abstand zwischen dem ersten und dem zweiten Mikrokanal allmählich der Länge davon entlang variiert.

11. Vorrichtung nach Anspruch 1, wobei die Variation des Abstands zwischen dem ersten und dem zweiten Mikrokanal der Länge davon entlang die Veränderung mindestens eines von einem chemischen oder einem mechanischen Reiz der Länge dieses Mikrokanals entlang ermöglicht.

12. Verfahren zum Züchten von Zellen, wobei das Verfahren Folgendes umfasst:
(a) das Bereitstellen eines Bioreaktors umfassend eine Vorrichtung nach einem der Ansprüche 1 bis 13;
(b) das Einführen von Zellen in mindestens einen der Mikrokanäle; und
(c) das Züchten der Zellen.

13. Verfahren nach Anspruch 12, ferner das Einführen eines Fluids in mindestens einen der Mikrokanäle umfassend.

14. Verfahren nach Anspruch 12, ferner das Aussetzen der Zellen mindestens einem mechanischen, einem chemischen oder einem biologischen Reiz und das Messen einer Änderung der Zellfunktion der Zellen auf den Reiz hin umfassend.

15. Verfahren nach Anspruch 12, wobei Schritt (b) Folgendes umfasst:
das Einführen verschiedener Typen von Zellen in den mindestens einen Mikrokanal;
das Einführen verschiedener Typen von Zellen in verschiedene Mikrokanäle; und
das Impfen verschiedener Typen von Zellen an verschiedenen ausgewählten Stellen innerhalb des mindestens einen Mikrokanals.

## Revendications

1. Dispositif bioréacteur microfluidique comprenant:
une première couche polymère définissant un premier et un second microcanal à l'intérieur et une seconde couche polymère définissant un troisième microcanal à l'intérieur; et
une membrane séparant le premier et le second canal du troisième canal à des parties géométriquement chevauchantes entre eux, la membrane permettant la communication entre les parties chevauchantes des microcanaux,
où une distance entre le premier et le second microcanaux varie le long d'une longueur de ceux-ci et une profondeur et une largeur du premier microcanal et une profondeur et une largeur du second microcanal demeurent constantes le long de leurs longueurs à l'intérieur des parties chevauchantes.

2. Dispositif selon la revendication 1 où le premier microcanal communique avec le second microcanal *via* le troisième microcanal.

3. Dispositif selon la revendication 1, où la membrane comprend au moins l'un parmi une nappe, un matériau électrofilé, un polydiméthylsiloxane micromoulé, une polyéthersulfone, ou une membrane à traces attaquées.

4. Dispositif selon la revendication 1, comprenant en outre au moins un type de cellule qui adhère à au moins l'une parmi : la membrane et une paroi des microcanaux.

5. Dispositif selon la revendication 4, où le type de cellule varie le long d'une longueur du microcanal.

6. Dispositif selon la revendication 1, comprenant en outre un liquide dans les microcanaux, le liquide étant constitué d'au moins l'un parmi un milieu de culture de cellules, une solution tampon, des composants sanguins, du sang total, de l'urine, un dialysat, de l'eau, un filtrat, ou une solution reproduisant un liquide corporel.

7. Dispositif selon la revendication 1, dans lequel une valeur de pression, de débit d'écoulement, de vitesse de cisaillement, ou de viscosité dans au moins l'un parmi le premier et le second microcanal est substantiellement différente d'une valeur de pression, de débit d'écoulement, de vitesse de cisaillement, ou de viscosité dans le troisième microcanal.

8. Dispositif selon la revendication 1, comprenant en outre un quatrième microcanal sur le même côté de la membrane que le troisième microcanal, une valeur d'un paramètre de mécanique des fluides dans le quatrième microcanal étant substantiellement différente d'une valeur d'un paramètre de mécanique des fluides dans le troisième microcanal.

9. Dispositif selon la revendication 1, où une concentration ou un gradient de concentration d'un constituant dans le fluide dans au moins l'un du premier et du second microcanaux est substantiellement différent(e) d'une concentration ou d'un gradient de concentration du constituant dans le fluide dans le troisième microcanal.

10. Dispositif selon la revendication 1, où la distance entre le premier et le second microcanal varie progressivement le long de leurs longueurs.

11. Dispositif selon la revendication 1, où la variation de la distance entre le premier et le second microcanal le long de leurs longueurs facilite la variation d'au moins l'un parmi un stimulus chimique ou un stimulus mécanique le long de la longueur de ce microcanal.

12. Procédé de culture de cellules, le procédé comprenant:
(a) la fourniture d'un bioréacteur comprenant un dispositif selon l'une quelconque des revendications 1 à 13;
(b) l'introduction de cellules dans au moins l'un des microcanaux; et
(c) la culture des cellules.

13. Procédé selon la revendication 12, comprenant en outre l'introduction d'un liquide à l'intérieur d'au moins l'un des microcanaux.

14. Procédé selon la revendication 12, comprenant en outre l'exposition des cellules à au moins l'un parmi un stimulus mécanique, un stimulus chimique, ou un stimulus biologique; et la mesure d'un changement dans la fonction cellulaire des cellules vis-à-vis du stimulus.

15. Procédé selon la revendication 12, où l'étape (b) comprend:
l'introduction de différents types de cellules dans le au moins un microcanal;
l'introduction de différents types de cellules dans différents microcanaux; et
l'ensemencement de différents types de cellules à différents emplacements sélectionnés à l'intérieur du au moins un microcanal.
